# EUROPÄISCHE PATENTSCHRIFT

(11) **EP 0 548 171 B1**
(45) Veröffentlichungstag und Bekanntmachung des Hinweises auf die Patenterteilung: **11.12.1996**
(21) Anmeldenummer: 91916264.4
(22) Anmeldetag: 12.09.1991
(51) Int. Cl.: A01N 53/00, A01N 25/22

(54) **INSEKTIZIDE ZUSAMMENSETZUNG**
INSECTICIDAL COMPOSITION
COMPOSITION INSECTICIDE

(30) Priorität: 12.09.1990 DE 9013000 U
(43) Veröffentlichungstag der Anmeldung: 30.06.1993
(73) Patentinhaber: PERYCUT-CHEMIE A.G., 8053 Zürich (CH); BENCSITS, Franz, CH-8053 Zürich (CH)
(72) Erfinder: BENCSITS, Franz, CH-8053 Zürich (CH)
(74) Vertreter: Grünecker, Kinkeldey, Stockmair & Schwanhäusser Anwaltssozietät
(86) Internationale Anmeldenummer: EP9101735
(87) Internationale Veröffentlichungsnummer: WO9203926

(56) Entgegenhaltungen:
- FR-A- 2 375 826
- GB-A- 625 683
- GB-A- 1 013 946
- US-A- 2 377 029
- US-A- 2 383 815
- US-A- 2 485 640
- US-A- 3 560 613
- US-A- 4 125 400
- JOURNAL OF THE SCIENCE OF FOOD AND AGRICULTURE, Bd. 3, Mai 1952, BARKING, GB, Seiten 219-224; R.E.BLACKITH: 'STABILITY OF CONTACT INSECTICIDES. I--Ultra-violet Photolysis of the Pyrethrins', siehe Seite 219, Zusammenfassung; Seiten 223-224, Abschnitt: "Conclusions"

## Beschreibung

Die vorliegende Erfindung betrifft eine insektizide Zusammensetzung umfassend eine wirksame Menge mindestens eines Pyrethroids, mindestens eines UV-Absorptionsmittles und mindestens eines Antioxidationsmittels, und seine Verwendung zur Bekämpfung von fliegenden und kriechenden Insekten.

Als Pyethroide werden die insektizid wirksamen Inhaltsstoffe des Pyrethrums sowie deren synthetische Analoga, die sich von der nachstehend angegebenen Struktur ableiten, bezeichnet. Die hauptsächlichen Wirkstoffe in Pyrethrum sind die Cinerine I und II, die Pyrethrine I und II und die Jasmoline I und II (Römpps Chemie-Lexikon, 8. Aufl. (1987), S. 3413). 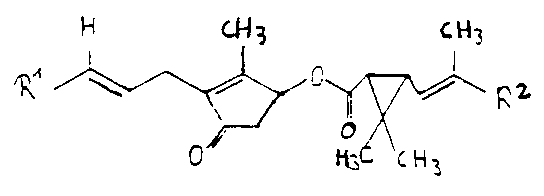
- Pyrethrin I:: R¹ = CH=CH₂, R² = CH₃
- Pyrethrin II:: R¹ = CH=CH₂, R² = COOCH₃
- Cinerin I:: R¹ = R² = CH₃
- Cinerin II:: R¹ = CH₃, R² = COOCH₃
- Jasmolin I:: R¹ = C₂H₅, R² = CH₃
- Jasmolin II:: R¹ = C₂H₅, R² = COOCH₃
- Allethrin:: R¹ = H, R² = CH₃

Pyrethrum wird aus den getrockneten Blütenköpfen verschiedener Pyrethrum- oder Chrysanthemum-Arten durch Pulverisieren oder Extraktion gewonnen und enthält als Hauptwirkstoffe Pyrethroide, wie Pyrethrine, Cinerine und Jasmoline. Neben Nikotin ist Pyrethrum das stärkste pflanzliche Insektizid; seine Wirksamkeit wird jedoch durch Sonnenlicht und Wärme verringert (Römpps Chemie-Lexikon, 8. Aufl. (1987), S. 3414). Der Mangel an Stabilität, aber auch der hohe Preis der natürlichen Pyrethroide hat zur Entwicklung zahlreicher synthetischer Derivate geführt.

Die Pyrethroide werden im allgemeinen als Isomerengemische eingesetzt. Sie werden seit langem als Insektenvernichtungsmittel verwendet, insbesondere gegen Stubenfliegen, Kakerlaken bzw. Schaben und anderes Hausungeziefer, Motten, Kornkäfer, Moskitos, Garten- und Gewächshausschädlinge, Heu- und Sauerwurm im Weinbau und Baumwollschädlinge. Besonders die natürlichen Pyrethroide zeichnen sich durch einen raschen sogenannten "Knock down-Effekt" aus, d.h. die Insekten sind zwar rasch, aber nur vorübergehend gelähmt und erholen sich wieder. Verantwortlich für diesen und unerwünschten Effekt ist der oxidative Entgiftungsstoffwechsel der Insekten.

Aufgrund der hohen Instabilität der Pyrethroide gegenüber Luft und Licht wurden bereits zahlreiche Versuche unternommen, Pyrethroide zu stabilisieren und ihre Wirksamkeit zu verlängern.

Pyrethrum Post 11(4), 135-7, 51 und J. Agr. Food Chem., 20(2), 313-15 offenbaren die Stabilisierung von Pyrethrinen und Allethrin durch Zusatz eines Antioxidationsmittels und eines UV-Absorptionsmittels in einer Mineralölformulierung. Während nichtstabilisierte Formulierungen innerhalb von 4 h fast vollständig zerstört sind, fuhrt die Kombination eines Oxidationsmittels und eines UV-Absorptionsmittels zu einer beträchtlichen Stabilisierung der Pyrethroide über wenigstens 4 h.

Als UV-Absorptionsmittel werden aromatische Ketone, in denen zwei aromatische Ringe direkt an eine Oxogruppe gebunden sind, z.B. Benzophenonderivate, und Ester von aromatischen Säuren, z.B. Ester von substituierten Benzoesäuren, genannt. Die verwendeten Antioxidationsmittel weisen eine OH-Gruppe, die direkt an einen aromatischen Ring gebunden ist, und wenigstens 14 Kohlenstoffatome auf, wie 4-Methyl-2,6-di-tert-butylphenol oder 2,6-Dioctadecyl-p-kresol.

Die US-A-3560613, WO-A-86/03374, EP-A-147947, DE-A-2615646 und GB-A-2058569 beschreiben verschiedene insektizide Zusammensetzungen auf Pyrethroidbasis, die zur Stabilisierung des Pyrethroids Antioxidationsmittel und übliche UV-Absorptionsmittel enthalten. Als Antioxidationsmittel werden 2,6-Di-tert.-butyl-4-methylphenol, 2,6-Dioctadecyl-p-cresol, butyliertes Hydroxytoluol, alkylierte Phenole, tert.-Butylhydrochinon, butylierte Hydroxyanisole, Ethoxychin, Ascorbinsäure, deren Salze und Derivate und Propionsäuresalze verwendet. Die Stabilität dieser Zusammensetzungen ist jedoch nicht zufriedenstellend.

Aufgabe der vorliegenden Erfindung ist es, eine insektizide Zusammensetzung mit verbesserter Langzeitwirkung zur Verfügung zu stellen.

Diese Aufgabe wird durch eine insektizide Zusammensetzung der eingangs genannten Art gelöst, die dadurch gekennzeichnet ist, daß das enthaltene Antioxidationsmittel mindestens ein Citronensäureester ist.

Die erfindungsgemäße Zusammensetzung eignet sich zur Bekämpfung von Ungeziefer, wie Hausungeziefer, insbesondere Fliegen und Kakerlaken bzw. Schaben.

Die erfindungsgemäße insektizide Zusammensetzung weist überraschenderweise eine stark verbesserte Langzeitwirkung gegen Ungeziefer auf aufgrund ihrer großen Stabilität gegenüber UV-Licht bzw. ihrer Oxidationsbeständigkeit. Die Empfindlichkeit der Zusammensetzung gegenüber UV-Licht ist auf eine Oxidation der freien Fettsäuren zurückzuführen, wobei auch eine Autoxidation durch eine chemische Reaktion des Luftsauerstoffs mit den in ungesättigten Fettsäuren enthaltenen Doppelbindungen möglich ist. Diese Empfindlichkeit wird erfindungsgemäß durch den Einsatz von Citronensäureestern als Antioxidationsmittel drastisch verringert. Als weiteres Antioxidationsmittel kann Ascorbylpalmitat enthalten sein.

Die Citronensäureester sind vorzugsweise Mono- bis Triester der Citronensäure mit Alkylalkoholen mit 1 bis 8 Kohlenstoffatomen.

Das Antioxidationsmittel wird in der erfindungsgemäßen Zusammensetzung vorzugsweise in einer Menge von 0,001 bis 10 Gew.-%, besonders bevorzugt 0,01 bis 5 Gew.-%, insbesondere 0,03 Gew.-%, bezogen auf die Zusammensetzung, verwendet.

In der erfindungsgemäßen insektiziden Zusammensetzung können alle natürlichen und synthetischen Pyrethroide, entweder allein oder in Mischung, verwendet werden. Als besonders wirksam hat sich dabei natürliches Pyrethrum erwiesen.

Das Pyrethroid wird vorzugsweise in einer Menge von 0,001 bis 10 Gew.-%, besonders bevorzugt 0,01 bis 2 Gew.-%, insbesondere 0,03 Gew.-%, bezogen auf die Zusammensetzung, verwendet.

Erfindungsgemäß verwendete UV-Absorptionsmittel sind allgemein bekannt. Besonders geeignete UV-Absorptionsmittel, die in dem hier interessierenden Wellenlängenbereich von 250 bis 350 nm wirksam sind, sind Benzoesäurederivate, beispielsweise p-Aminobenzoesäurederivate, wie Amyl-p-dimethylaminobenzoat und Glyceryl-p-aminobenzoat, oder o-Hydroxybenzoesäurederivate; Benzophenonderivate, beispielsweise 2-Hydroxy-4-(2-hydroxy-3-methacryloxy)-propoxybenzophenon oder 2-Hydroxy-4-methoxybenzophenon-5-sulfonsäure; Campferderivate; Cumarinderivate; Benzimidoazolderivate; Dibenzoylmethanderivate; Zimtsäureesterderivate, beispielsweise Isobutylzinnamat, Ethylzinnamat oder Benzylzinnamat; und Tris(hydroxymethyl)-aminomethansalze einer Sulfonsäure, wie das Tris(hydroxymethyl)aminomethansalz der 2-Phenylbenzimidazol-5-sulfonsäure.

Das UV-Absorptionsmittel wird vorzugsweise in einer Menge von 0,001 bis 10 Gew.-%, besonders bevorzugt 0,01 bis 5 Gew.-%, insbesondere 0,03 Gew.-%, bezogen auf die Zusammensetzung, verwendet.

Zweckmäßigerweise wird die erfindungsgemäße insektizide Zusammensetzung in flüssiger Form unter Zusatz eines Verdünnungs- bzw. Lösungsmittels oder in Pulverform hergestellt. Geeignete Verdünnungsmittel sind Wasser, organische Lösungsmittel oder Öle, wobei eine wäßrige und/oder ölige Emulsion bevorzugt ist. Als organische Verdünnungsmittel können beispielsweise ein und mehrwertige Alkohole, Glykole, wie 1,2-Propandiol, und halogenierte Kohlenwasserstoffe verwendet werden, während als Öle gesättigte und ungesättigte Wachs- und Fettsäureester, beispeilsweise Pflanzenöle, sowie natürliche und synthetische etherische Öle besonders gut geeignet sind. Zusätzlich kann die erfindungsgemäße insektizide Zusammensetzung Lecithin enthalten. Bei der praktischen Anwendung kann die insektizide Zusammensetzung dann auf die zu behandelnde Fläche aufgesprüht werden.

Die Zubereitung der erfindungsgemäßen insektiziden Zusammensetzung in Pulverform erfolgt auf übliche Weise unter Zusatz eines festen Trägers, wie Talkum, Zink- und Titandioxid, gebranntes Magnesiumoxid oder eines wasserfreien Metallsalzes.

Die erfindungsgemäße Zusammensetzung kann für jede Art von Ungeziefer, gegen die bereits bekannte Pyrethroidzusammensetzungen verwendet werden, eingesetzt werden, insbesondere zur Vernichtung von Hausungeziefer, wie Fliegen und Kakerlaken.

Die erfindungsgemäße insektizide Zusammensetzung besitzt eine ausgezeichnete Langzeitwirkung gegen fliegende Insekten, beispielsweise Stubenfliegen und Motten, und kriechende Insekten, beispielsweise Kakerlaken und Ameisen.

## Patentansprüche

1. Insektizide Zusammensetzung, umfassend eine wirksame Menge mindestens eines Pyrethroids, mindestens eines UV-Absorptionsmittels und mindestens eines Antioxidationsmittels, **dadurch gekennzeichnet,** daß das Antioxidationsmittel mindestens ein Citronensäureester ist.

2. Zusammensetzung nach Anspruch 1, **dadurch gekennzeichnet,** daß das Pyrethroid Pyrethrum ist.

3. Zusammensetzung nach Anspruch 1 oder 2, **dadurch gekennzeichnet,** daß das UV-Absorptionsmittel ein Benzoesäurederivat, ein Benzophenonderivat, ein Benzoxazolderivat, ein Campherderivat, ein Cumarinderivat, ein Benzimidazolderivat, ein Dibenzoylmethanderivat, ein Zimtsäureesterderivat oder ein Tris(hydroxymethyl)aminomethansalz einer Sulfonsäure ist.

4. Zusammensetzung nach einem der Ansprüche 1 bis 3, **dadurch gekennzeichnet,** daß das UV-Absorptionsmittel ein p-Aminobenzoesäureaerivat ist.

5. Zusammensetzung nach einem der Ansprüche 1 bis 4, **dadurch gekennzeichnet,** daß sie 0,001 bis 10 Gew.-% des Pyrethroids enthält.

6. Zusammensetzung nach Anspruch 5, **dadurch gekennzeichnet,** daß sie 0,01 bis 2 Gew.-% des Pyrethroids enthält.

7. Zusammensetzung nach einem der Ansprüche 1 bis 6, **dadurch gekennzeichnet,** daß sie 0,001 bis 10 Gew.-% des UV-Absorptionsmittels enthält.

8. Zusammensetzung nach Anspruch 7, **dadurch** **gekennzeichnet,** daß sie 0,01 bis 5 Gew.-% des UV-Absorptionsmittels enthält.

9. Zusammensetzung nach einem der Ansprüche 1 bis 8, **dadurch gekennzeichnet,** daß sie 0,001 bis 10 Gew.-% des Antioxidationsmittels enthält.

10. Zusammensetzung nach Anspruch 9, **dadurch gekennzeichnet,** daß sie 0,01 bis 5 Gew.-% des Antioxidationsmittels enthält.

11. Zusammensetzung nach einem der Ansprüche 1 bis 10, **dadurch gekennzeichnet,** daß sie als weiteres Antioxidationsmittel Ascorbylpalmitat enthält.

12. Zusammensetzung nach einem der Ansprüche 1 bis 11, **dadurch gekennzeichnet,** daß sie übliche Träger und/oder Verdünnungsmittel enthält.

13. Zusammensetzung nach einem der Ansprüche 1 bis 12, **dadurch gekennzeichnet,** daß sie in wäßriger und/oder öliger Emulsion vorliegt.

14. Zusammensetzung nach einem der Ansprüche 1 bis 13, **dadurch gekennzeichnet,** daß sie zusätzlich Lecithin enthält.

15. Verwendung der insektiziden Zusammensetzung nach einem der Ansprüche 1 bis 14 zur Bekämpfung von fliegenden und kriechenden Insekten, insbesondere von Fliegen und Kakerlaken.

## Claims

1. Insecticidal composition, containing an active quantity of at least one pyrethroid, at least one UV absorber and at least one antioxidant, characterised in that the antioxidant is at least one citric ester.

2. Composition according to claim 1, characterised in that the pyrethroid is pyrethrum.

3. Composition according to claim 1 or 2, characterised in that the UV absorber is a benzoic acid derivative, a benzophenone derivative, a benzoxazole derivative, a camphor derivative, a coumarin derivative, a benzimidazole derivative, a dibenzoylmethane derivative, cinnamic ester derivative or a tris(hydroxymethyl)aminomethane salt of a sulphonic acid.

4. Composition according to one of claims 1 to 3, characterised in that the UV absorber is a p-aminobenzoic acid derivative.

5. Composition according to one of claims 1 to 4, characterised in that it contains from 0.001 to 10 wt.% of the pyrethroid.

6. Composition according to claim 5, characterised in that it contains from 0.01 to 2 wt.% of the pyrethroid.

7. Composition according to one of claims 1 to 6, characterised in that it contains from 0.001 to 10 wt.% of the UV absorber.

8. Composition according to claim 7, characterised in that it contains from 0.01 to 5 wt.% of the UV absorber.

9. Composition according to one of claims 1 to 8, characterised in that it contains from 0.001 to 10 wt.% of the antioxidant.

10. Composition according to claim 9, characterised in that it contains from 0.01 to 5 wt.% of the antioxidant.

11. Composition according to one of claims 1 to 10, characterised in that it contains as an additional antioxidant ascorbyl palmitate.

12. Composition according to one of claims 1 to 11, characterised in that it contains conventional carriers and/or diluents.

13. Composition according to one of claims 1 to 12, characterised in that it is in aqueous and/or oleaginous emulsion.

14. Composition according to one of claims 1 to 13, characterised in that it additionally contains lecithin.

15. Use of the insecticidal composition according to one of claims 1 to 14 for the control of flying and crawling insects, in particular flies and cockroaches.

## Revendications

1. Composition insecticide contenant une quantité active d'au moins un pyréthroïde, au moins un agent absorbant les UV et au moins un antioxydant, **caractérisée en ce que** l'antioxydant est au moins un ester de l'acide citrique.

2. Composition selon la revendication 1, **caractérisée en ce que** le pyréthroïde est du pyrèthre.

3. Composition selon la revendication 1 ou 2, **caractérisée en ce que** l'agent absorbant les UV est un dérivé de l'acide benzoïque, un dérivé de benzophénone, un dérivé du benzoxazole, un dérivé du camphre, un dérivé de la coumarine, un dérivé du benzimidazole, un dérivé du dibenzoylméthane, un dérivé d'ester de l'acide cinnamique ou un sel du Tris(hydroxyméthyl)aminométhane d'un acide sulfonique.

4. Composition selon l'une quelconque des revendications 1 à 3, **caractérisée en ce que** l'agent absorbant les UV est un dérivé d'acide p-aminobenzoïque.

5. Composition selon l'une quelconque des revendications 1 à 4, **caractérisée en ce qu**'elle contient 0,001 à 10 % en poids du pyréthroïde.

6. Composition selon la revendication 5, **caractérisée en ce qu**'elle contient 0,01 à 2 % en poids du pyréthroïde.

7. Composition selon l'une quelconque des revendications 1 à 6, **caractérisée en ce qu**'elle contient 0,001 à 10 % en poids de l'agent absorbant les UV.

8. Composition selon la revendication 7, **caractérisée en ce qu**'elle contient 0,01 à 5 % en poids de l'agent absorbant les UV.

9. Composition selon l'une quelconque des revendications 1 à 8, **caractérisée en ce qu**'elle contient 0,001 à 10 % en poids de l'antioxydant.

10. Composition selon la revendication 9, **caractérisée en ce qu**'elle contient 0,01 à 5 % en poids de l'antioxydant.

11. Composition selon l'une quelconque des revendications 1 à 10, **caractérisée en ce qu**'elle contient du palmitate d'ascorbyle en tant qu'autre antioxydant.

12. Composition selon l'une quelconque des revendications 1 à 11, **caractérisée en ce qu**'elle contient des supports et/ou diluants habituels.

13. Composition selon l'une quelconque des revendications 1 à 12, **caractérisée en ce qu**'elle se présente sous la forme d'une émulsion aqueuse et/ou huileuse.

14. Composition selon l'une quelconque des revendications 1 à 13, **caractérisée en ce qu**'elle contient en outre de la lécithine.

15. Utilisation de la composition insecticide selon l'une quelconque des revendications 1 à 14 pour combattre des insectes volants et rampants, notamment des mouches et des cancrelats.
